# EUROPEAN PATENT APPLICATION

(11) **EP 2 407 164 A1**
(43) Date of publication of application: **18.01.2012**
(21) Application number: 10169467.7
(22) Date of filing: 14.07.2010
(51) Int. Cl.: A61K 31/30, C07F 1/08, A61P 35/00

(54) **Copper II complexes of phenanthroline and their use in cancer treatment**

(71) Applicant: Dublin Institute of Technology Intellectual Property Ltd, D2 Dublin (IE)
(72) Inventor: The designation of the inventor has not yet been filed
(74) Representative: Purdy, Hugh Barry

(57) **Abstract**

The invention relates to a Cu^{II} complex in which the copper ion is coordinated to an aromatic, non-aromatic or aliphatic dicarboxylate ligand via an oxygen atom of one of the carboxylate groups, and two phenanthroline, or phenanthroline derivative, ligands, and the use of the metal complexes as self-activating nucleases to treat cancer and other proliferative disorders. In one embodiment, the Cu^{II} complex is a dinuclear complex comprising two Cu^{II} ions, in which the aromatic, non-aromatic or aliphatic dicarboxylate ligand is a bridging ligand, and in which each copper is coordinated to the bridging ligand via the carboxylate groups.

## Description

### Technical Field

The invention relates to a copper²⁺ complexes of phenanthroline. In particular, the invention relates to copper²⁺ complexes of phenanthroline that exhibit self-activating nuclease activity *in-vivo.*

### Background to the Invention

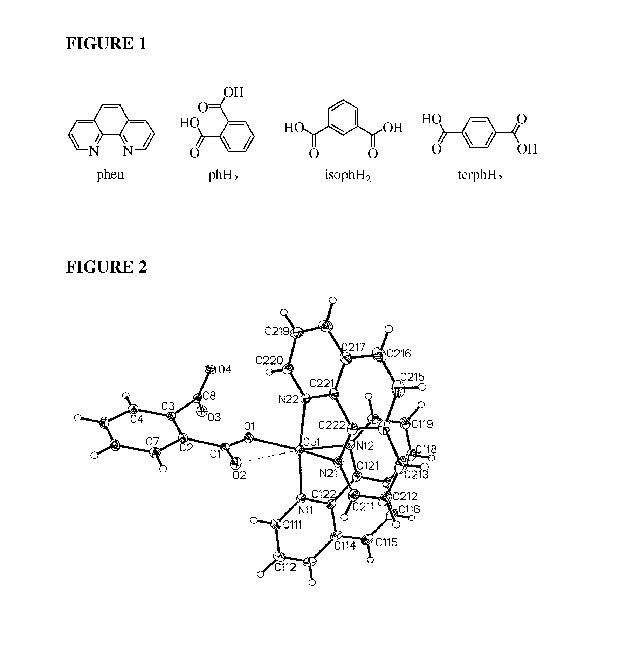

The design of agents capable of controlled cleavage of DNA and RNA is of paramount importance due to their potential as DNA-targeted chemotherapeutic drugs and their potential applications in biology. Complexes of metals such as iron, copper and manganese have been shown to act as effective chemical nucleases through oxidative induced DNA damage. The oxidative DNA cleavage activity of Cu²⁺ complexes of 1,10-phenanthroline (phen, ***Figure 1***) in the presence of the co-reactant H₂O₂ was reported by Sigman et al. in 1979 and the activity involves a succession of redox events with a copper-`oxo' species believed to be the active agent.¹ More recently the ligands 2-clip-phen and 3-clip-phen were investigated as systems that maintain the 2:1 phen-Cu stoichiometry at low concentrations and the oxidative nuclease activity of copper complexes of these new ligands was found to be higher than that of phen itself and it would appear they induce a different DNA oxidation profile to the parent [Cu(phen)₂]²⁺.^{2, 3} The DNA cleavage reactions of the copper bis-phen and the copper clip-phen systems must be initiated by an excess of an exogeneous agent such as H₂O₂, and/or an appropriate reducing agent, which limits their *in vivo* applications. Only a few self-activating DNA cleavage systems have been reported including Fe(BLM) and Cu(BLM) (BLM = bleomycin) which require the presence of molecular oxygen.³ Bis(hydroxysalicylidene)ethylenediamine, Cu-tambjamine E and its pyrrolidine derivatives, Hypyramol and 2-(2-pyridyl)benzthiazole have all been reported to cleave DNA in the presence of copper(II) and molecular oxygen and a number of them exhibit anticancer activity.⁴⁻⁷ To our knowledge no copper bis-phen complexes have been reported to cleave DNA in the absence of added reductant. Karlin and co-workers and recently Pitié and Reedijk have demonstrated that nuclearity is an important factor in oxidative DNA cleavage.^{8,9} Copper bis-phen systems are among a relatively small group of metallo-systems can bind DNA efficiently and selectively activate C-H deoxyribose bonds leading to strand scission.

Phen and its substituted derivatives have high affinities for copper ions¹⁰⁻¹² and copper complexes of phen have been shown to exhibit excellent antitumour¹³, anti*-candida*¹⁴ and antibacterial¹⁵ activities. The tumor suppressor gene p53 is known to become mutated in many human cancers and thus fails to function. It has been demonstrated that phen enhances p53 activity *in vitro* and can trigger apoptosis in p53 negative cell lines^{16, 17}.

### Statements of Invention

The invention relates to a Cu^{II} complex in which the copper ion is coordinated to an aromatic, non-aromatic or aliphatic dicarboxylate ligand via an oxygen atom of one of the carboxylate groups, and two phenanthroline, or phenanthroline derivative, ligands, and the use of the metal complexes as self-activating nucleases to treat cancer and other proliferative disorders. In one embodiment, the Cu^{II} complex is a dinuclear complex comprising two Cu^{II} ions, in which the aromatic, non-aromatic or aliphatic dicarboxylate ligand is a bridging ligand, and in which each copper is coordinated to the bridging ligand via the carboxylate groups.

In a first aspect, the invention relates to a method of treating a cancer comprising a step of treating an individual with a therapeutically effective amount of a metal complex having a general structure I:

**I** [Y₂-X-Y₁]ⁿ⁺ • B

in which:
- X is an optionally substituted, branched or unbranched, aliphatic dicarboxylic acid, or a monocyclic or polycyclic aromatic, non-aromatic, or heterocyclic, dicarboxylic acid;
- Y₁ is Cu²⁺(Phen)₂L₁ having the structure: 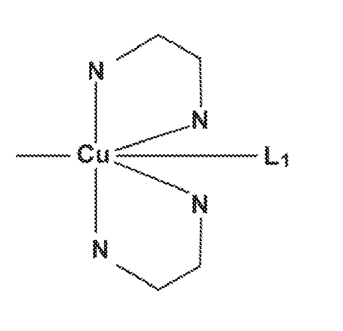 in which: 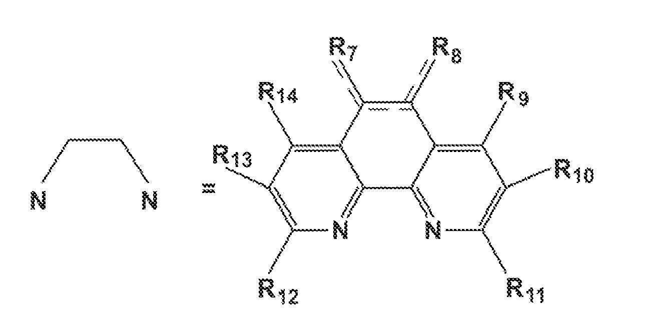
   Cu²⁺ is coordinated to an oxygen atom of a carboxylate group of X;
   R₇ to R₁₄ are each, independently, selected from oxygen, hydrogen, hydroxyl, a carboxyl, a substituted or unsubstituted, branched or unbranched alkyl, alkenyl, cycloalkyl, aryl, aryloxy, alkyloxy, arylalkyloxy, amino, alkylamino, hydroxylamino, dialkylamino, or alkoxy group, or pyridine, or are fused to form a monocyclic or polycyclic aromatic hydrocarbon or heterocycle;
   L₁ is absent or is a unidentate ligand; and
   a broken line designates a single or double bond,
- Y₂ is absent, or when present is Cu²⁺(Phen)₂L₁ in which the copper ion is coordinated to an oxygen atom of a carboxylate group of X;
- n⁺ is the charge on the complex ion or is absent; and
- B is a counterion or is absent.

The invention also relates to the use of the metal complex of general formula II as a self-activating metallonuclease.

In one embodiment of the method of the invention, X is an optionally substituted, branched or unbranched, aliphatic dicarboxylic acid. Typically, X has a formula COO⁻-(CH₂)ₙ-COO⁻, in which n is 2 to 20. In a preferred embodiment, n is 3 to 10. Ideally, n is 8 in which case X is suberic acid.

Suitably, the metal complex is a mono-nuclear Cu²⁺ complex having a formula: 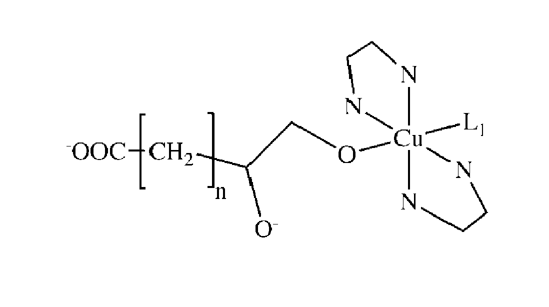 in which:
- n = 0 to 20.
Depending on the aliphatic chain length, the unidentate ligand could comprise the free carboxylate of X.

Typically, the metal complex is a di-nuclear Cu²⁺ complex having a formula:

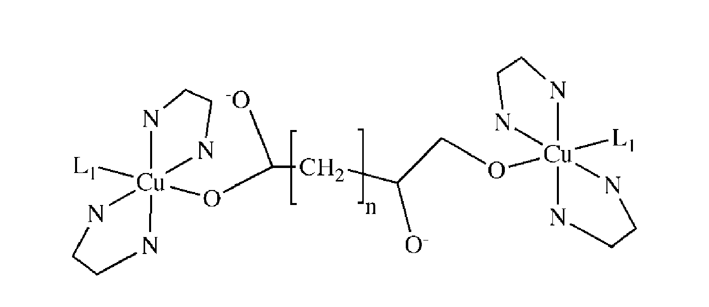

In another embodiment of the method of the invention, X is an optionally substituted aromatic dicarboxylic acid. Suitable examples of aromatic dicarboxylic acids are phthalic acid, phthalic acid isomers (for example isophthalic acid, terephthalic acid) and derivatives thereof.

Typically, the metal complex has a general structure II: 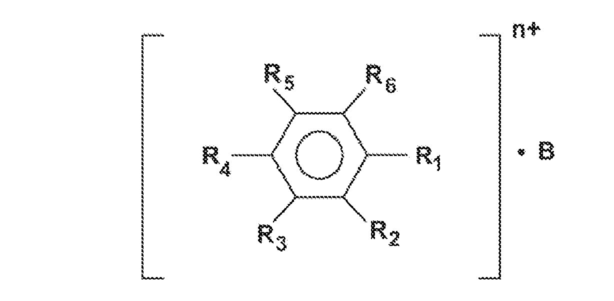 in which:
R₁ to R₆ are each, independently, selected from oxygen, hydrogen, hydroxyl, a carboxyl, a substituted or unsubstituted, branched or unbranched alkyl, alkenyl, cycloalkyl, aryl, aryloxy, alkyloxy, arylalkyloxy, amino, alkylamino, hydroxylamino, dialkylamino, or alkoxy group, or pyridine, or are fused to form a monocyclic or polycyclic aromatic hydrocarbon or heterocycle, or is a substituent
   -Z-COO⁻-Cu²⁺(Phen)₂ having the formula: 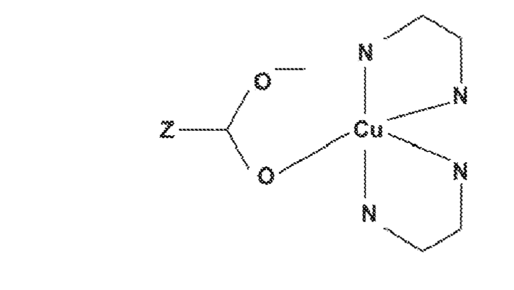 in which: 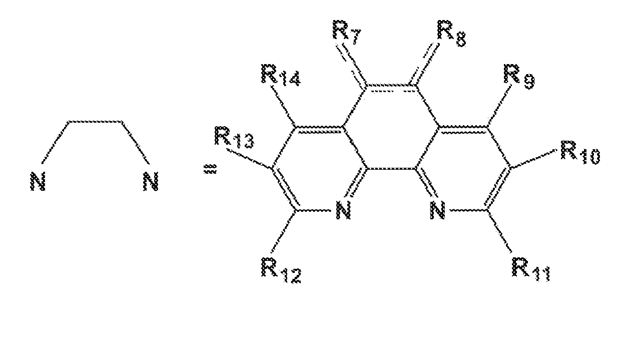
   R₇ to R₁₄ are each, independently, selected from oxygen, hydrogen, hydroxyl, a carboxyl, a substituted or unsubstituted, branched or unbranched alkyl, alkenyl, cycloalkyl, aryl, aryloxy, alkyloxy, arylalkyloxy, amino, alkylamino, hydroxylamino, dialkylamino, or alkoxy group, or pyridine, or are fused to form a monocyclic or polycyclic aromatic hydrocarbon or heterocycle;
   a broken line designates a single or double bond; and
   Z is a spacer group or is absent,
- n+ is the charge on the complex ion, or is absent; and
B is a counterion or is absent,
wherein one or two of R₁ to R₆ is -Z-COO⁻-Cu²⁺(Phen)₂, and wherein when one of R₁ to R₆ is -Z-COO⁻-Cu²⁺(Phen)₂, another of R₁ to R₆ is a carboxylate substituent.

In a preferred embodiment of the method of the invention, n+ is 2+, and the counterion is phthalic acid, or a derivative thereof.

Typically, each phenanthroline ligand, independently, comprises 1,10 phenanthroline. The term "(phen)₂" as used herein should also be understood to include 2- or 3- clip-phen molecules^{3,5}.

The metal complex of the invention is either a mono-nuclear Cu²⁺ complex, which generally has a neutral charge and in which the counterion B is therefore absent, or is a bi-nuclear Cu²⁺ complex having two copper ions, each coordinated to two phenanthroline (or phenanthroline derivative) ligands, and an aromatic dicarboxylate bridging ligand, which will generally be charged and will ideally include a phthalate (or phthalate derivative) counterion.

In a preferred embodiment of the method of the invention, the metal complex is a mono-nuclear Cu²⁺ complex in which one of R₁ to R₆ is -Z-COO⁻-Cu²⁺(Phen)₂, one of R₁ to R₆ is a carboxylate group, and the remainder of R₁ to R₆ are typically H. This, the following mono-nuclear metal complexes are provided:
- R₁ is -Z-COO⁻-Cu²⁺(Phen)₂, R₂ is a carboxylate, and R₃, R₄, R₅ and R₆ are ideally H;
- R₁ is -Z-COO⁻-Cu²⁺(Phen)₂, R₃ is a carboxylate, and R₂, R₄, R₅ and R₆ are ideally H;
- R₁ is -Z-COO⁻-Cu²⁺⁽Phen)₂, R₄ is a carboxylate, and R₂, R₃, R₅ and R₆ are ideally H;
- R₁ is -Z-COO⁻-Cu²⁺(Phen)₂, R₅ is a carboxylate, and R₂, R₃, R₄ and R₆ are ideally H; and
- R₁ is -Z-COO⁻-Cu²⁺(Phen)₂, R₆ is a carboxylate, and R₂, R₃, R₄ and R₅ are H;

In the above complexes, the aromatic dicarboxylate ligand is phthalic acid, or a phthalic acid derivative. Generally, the spacer Z is absent. Typically, the non-carboxylate substituents are H.

In a another embodiment of the method of the invention, the metal complex is a dinuclear Cu^{II} complex in which two of R₁ to R₆ are -Z-COO⁻-Cu²⁺(Phen)₂ substituents, the remainder of R₁ to R₆ are typically H, and the counterion B is suitably a phthalic acid or phthalic acid derivative. Thus, the following di-nuclear metal complexes are provided:
- R₁ and R₂ are -Z-COO⁻-Cu²⁺(Phen)₂, and R₃, R₄, R₅ and R₆ are ideally H;
- R₁ and R₃ are -Z-COO⁻-Cu²⁺(Phen)₂, and R₂, R₄, R₅ and R₆ are ideally H;
- R₁ and R₄ are -Z-COO⁻-Cu²⁺(Phen)₂, and R₂, R₃, R₅ and R₆ are ideally H;
- R₁ and R₅ are -Z-COO⁻-Cu²⁺(Phen)₂, and R₂, R₃, R₄ and R₆ are ideally H; and
- R₁ and R₆ are -Z-COO⁻-Cu²⁺(Phen)₂, and R₂, R₃, R₄ and R₅ are ideally H;

In the above complexes, the bridging ligand is phthalic acid, or a phthalic acid derivative of the type having two carboxylate groups, in which the each of the carboxylate groups is coordinated to a CuII ion. Generally, the spacer Z is absent. Typically, the non-carboxylate substituents are H.

In one embodiment of the method of the invention, the metal complex contains a moiety which is capable of targeting a polynucleic acid sequence. The targeting moiety is generally designed to target the metal complex to a locus in a cell containing a genetic abnormality, for example a mutation or polymorphism typically associated with disease or disorder. Thus, when the locus of the abnormality to be targeted is DNA, the targeting moeity will be a DNA sequence capable of binding to the DNA at or adjacent to the abnormality. For example, the targeting moiety may be a nucleotide sequence, for example 5-50 nucleobases, suitably 5 to 20 nucleobases, having a sequence which has a binding affinity to a specific target sequence in the hosts nucleic acid sequences. Treating an individual with a metal complex of the invention which has been derivatised with such a targeting moiety will have the effect of concentrating the metal complex of the invention at sites in the body where the metal complex can cut the affected DNA. This technique can also be employed to treat other, non-proliferative disorders, in which a pathology is caused by undesirable mutations in DNA, for example degenerative conditions, autoimmune conditions, cardiovascular conditions and the like. Additionally, the targeting moiety can be designed to target different nucleotide molecules, for example mRNA, rRNA, microRNA, and the like. Generally, the targeting moiety will be covalently bound to one of the R₁ to R₁₄ groups of the metal complex, and techniques for derivatising the metal complex with a targeting moiety will be well known to those skilled in the art. Thus, the invention relates to the use of a metal complex of the type comprising a targeting moiety in the treatment of a disease or disorder characterized by a genetic abnormality, for example a proliferative disorder.

Suitably, the counterion, when present, is a pharmaceutically acceptable ion, for example an anion, typically selected from a chlorate, perchlorate, sulphate, thiosulphate, oxalate, carbonate, and phthalate or phthalate isomer, or a cation, the details of which will be well known to those skilled in the art. Ideally, the anion is selected from a phthalate or a phthalate isomer (isophthalate or terephthalate).

In a second aspect, the invention relates to a metal complex having a general structure II: 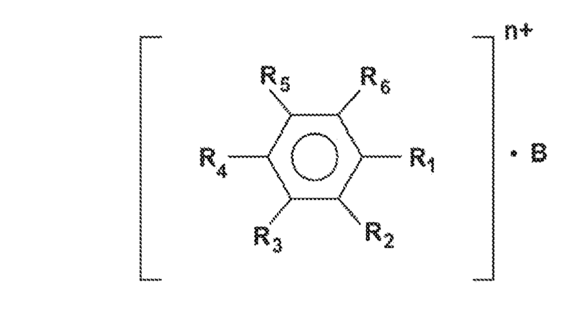 in which:
in which R₁ to R₆, -Z-COO⁻-Cu²⁺(Phen)₂, Z, n+ are as defined above,
and B is a counterion or is absent, with the proviso that B is not 2ClO₄⁻ ,
wherein one or two of R₁ to R₆ is -Z-COO⁻-Cu²⁺(Phen)₂, and wherein when one of R₁ to R₆ is -Z-COO⁻-Cu²⁺(Phen)₂, another of R₁ to R₆ is a carboxylate substituent.

In a preferred embodiment of the method of the invention, n+ is 2+, and the counterion is phthalic acid, or a derivative thereof.

In a preferred embodiment of the second aspect of the invention, the metal complex is a mono-nuclear Cu²⁺ complex in which one of R₁ to R₆ is -Z-COO⁻-Cu²⁺(Phen)₂, one of R₁ to R₆ is a carboxylate group, and the remainder of R₁ to R₆ are typically H. This, the following mono-nuclear metal complexes are provided:
- R₁ is -Z-COO⁻-Cu²⁺(Phen)₂, R₂ is a carboxylate, and R₃, R₄, R₅ and R₆ are ideally H;
- R₁ is -Z-COO⁻-Cu²⁺(Phen)₂, R₃ is a carboxylate, and R₂, R₄, R₅ and R₆ are ideally H;
- R₁ is -Z-COO⁻-Cu²⁺(Phen)₂, R₄ is a carboxylate, and R₂, R₃, R₅ and R₆ are ideally H;
- R₁ is -Z-COO⁻-Cu²⁺(Phen)₂, R₅ is a carboxylate, and R₂, R₃, R₄ and R₆ are ideally H; and
- R₁ is -Z-COO⁻-Cu²⁺(Phen)₂, R₆ is a carboxylate, and R₂, R₃, R₄ and R₅ are H;

In the above complexes, the aromatic dicarboxylate ligand is phthalic acid, or a phthalic acid derivative. Generally, the spacer Z is absent. Typically, the non-carboxylate substituents are H.

In a another embodiment of the method of the invention, the metal complex is a dinuclear Cu^{II} complex in which two of R₁ to R₆ are -Z-COO⁻-Cu²⁺(Phen)₂ substituents, the remainder of R₁ to R₆ are typically H, and the counterion B is suitably a phthalic acid or phthalic acid derivative. Thus, the following di-nuclear metal complexes are provided:
- R₁ and R₂ are -Z-COO⁻-Cu²⁺(Phen)₂, and R₃, R₄, R₅ and R₆ are ideally H;
- R₁ and R₃ are -Z-COO⁻-Cu²⁺(Phen)₂, and R₂, R₄, R₅ and R₆ are ideally H;
- R₁ and R₄ are -Z-COO⁻-Cu²⁺(Phen)₂, and R₂, R₃, R₅ and R₆ are ideally H;
- R₁ and R₅ are -Z-COO⁻-Cu²⁺(Phen)₂, and R₂, R₃, R₄ and R₆ are ideally H; and
- R₁ and R₆ are -Z-COO⁻-Cu²⁺(Phen)₂, and R₂, R₃, R₄ and R₅ are ideally H;

The invention thus relates to the use in treating cancer, or a proliferative disorder, of a Cu²⁺ complex in which the copper ion is coordinated to an aromatic dicarboxylate ligand via an oxygen atom of one of the carboxylate groups, and two phenanthroline, or phenanthroline derivative, ligands. In one embodiment, the Cu²⁺ complex is a dinuclear complex comprising two Cu²⁺ ions, in which the aromatic dicarboxylate ligand is a bridging ligand, and in which each copper is coordinated to the bridging ligand via the carboxylate groups.

Suitably, the metal complex of the invention is capable of exhibiting self-activating nuclease activity *in-vivo* (in other words, the metal complex has nuclease activity without the requirement for a co-factor). Preferably, the metal complex of the invention has a cytotoxicity of less than 100µM, 75µM, 50µM, 40µM, 30µM, 20µM, 15µM or 10µM against a cancer cell model selected from one or more of MCF-7 cells (breast cancer), DU145 cells (prostate cancer model), SKOV cells (ovarian cancer), and HT29 cells (colorectal cancer model). As used herein, the term "cytotoxicity" should be understood to mean the LD₅₀ (µM) over a period of 24 hours.

The invention also relates to a metal complex of the invention comprising a targeting ligand. The term targeting ligand should be understood to mean a moiety which is capable of targeting the metal complex of the invention to a specific site in the body. For example, the targeting ligand may be a nucleotide sequence, for example 5-50 nucleobases, suitably 5 to 20 nucleobases, having a sequence which has a binding affinity to a specific target sequence in the hosts nucleic acid sequences. For example, the targeting nucleotide sequence may be designed to target the metal complex to portion of the hosts DNA that includes an undesirable mutation or polymorphism. Such mutations or polymorphisms occur in patients having proliferative disorders, in which the mutation or polymorphism causes uncontrolled proliferation in affected cells or tissue. Treating an individual with a metal complex of the invention which has been derivatised with such a targeting ligand will have the effect of concentrating the metal complex of the invention at sites in the body where the metal complex can cut the affected DNA. This technique can also be employed to treat other, non-proliferative disorders, in which a pathology is caused by undesirable mutations in DNA, for example degenerative conditions, autoimmune conditions, cardiovascular conditions and the like. Additionally, the targeting ligand can be designed to target different nucleotide molecules, for example mRNA, rRNA, microRNA, and the like. Generally, the targetting ligand will be covalently bound to one of the R1 to R6 groups of the metal complex, and techniques for derivatising the metal complex of the invention with a targeting ligand will be well known to those skilled in the art.

The invention also relates to a method of treating a cancer comprising a step of treating an individual with a therapeutically effective amount of a metal complex of the invention, optionally in combination with another anti-cancer agent, for example Cisplatin, a HDAC inhibitor, a pyrimidine analogue, or the like.

The invention also relates to a method of treating a cell to inhibit proliferation of the cell comprising a step of treating the cell with a therapeutically effective amount of a metal complex of the invention, optionally in combination with another anti-cancer agent, for example Cisplatin, a HDAC inhibitor, a pyrimidine analogue, or the like.

The invention also relates to a method of treating a cancer comprising a step of treating an individual with a therapeutically effective amount of a metal complex of the invention, wherein the metal complex exhibits self-activating nuclease activity *in-vivo* (i.e. without the requirement for a co-factor).

The invention also provides a pharmaceutical composition comprising a therapeutically **effective** amount of a metal complex of the invention and a pharmaceutically acceptable carrier, optionally in combination with another anti-cancer agent, for example Cisplatin, a HDAC inhibitor, a pyrimidine analogue, or the like.

The invention relates to the use of a metal complex of the invention as a medicament.

### Brief Description of the Figures

***Figure 1******:*** Molecular structure of the phthalates and phen
***Figure 2*****:** X-Ray structure of [Cu(ph)(phen)₂] **(4)**

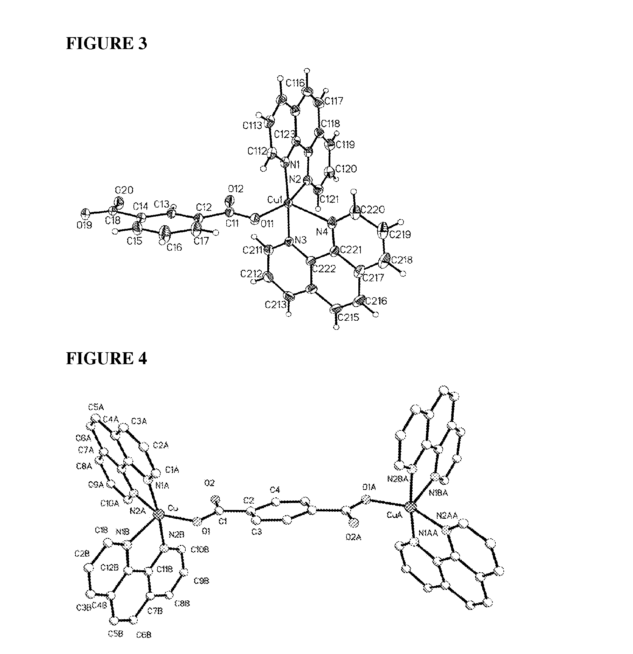

***Figure 3*****:** X-Ray structure of [Cu(isoph)(phen)₂] **(5)**
***Figure 4******:*** X-Ray structure of the cation in [{ Cu(phen)₂}₂(terph)](terph) **(6)**
***Figure 5*****:** Competitive EtBr displacement for actinomycin D, pentamidine and complexes **4-6** with CT-DNA
***Figure 6******:*** Relaxation of pUC18 by **4-6.** Reaction mixtures (20 µL total volume) contained 12.5 ng of supercoiled (Form I) DNA in 100 m*M* cacodylate buffer, pH 6. Cleavage was carried out at 37 °C for 5 hr and then analyzed by agarose gel electrophoresis. Lane 1: DNA alone; lanes 2-6: 5, 10, 20, 50, 100 µ*M* **4;** lanes 7-10: 10, 20, 50, 100 µ*M **5;*** lane 11-16: 1, 5, 10, 20, 50, 100 µ*M* **6.**
***Figure* 7****:** Competitive ethidium bromide displacement for complex **7** with CT-DNA
***Figure* 8****:** Relaxation of pUC18 by complex **7.** Cleavage was carried out at 37 °C for 5 hours and then analyzed by agarose gel electrophoresis.^{†} Lane 1: DNA alone; lanes 2-6: 1, 5, 10, 50, 100 µ*M* complex **7**

### Detailed Description of the Invention

In this specification, the term "cancer" should be taken to mean a cancer selected from the group consisting of: fibrosarcoma; myxosarcoma; liposarcoma; chondrosarcom; osteogenic sarcoma; chordoma; angiosarcoma; endotheliosarcoma; lymphangiosarcoma; lymphangioendotheliosarcoma; synovioma; mesothelioma; Ewing's tumor; leiomyosarcoma; rhabdomyosarcoma; colon carcinoma; pancreatic cancer; breast cancer; ovarian cancer; prostate cancer; squamous cell carcinoma; basal cell carcinoma; adenocarcinoma; sweat gland carcinoma; sebaceous gland carcinoma; papillary carcinoma; papillary adenocarcinomas; cystadenocarcinoma; medullary carcinoma; bronchogenic carcinoma; renal cell carcinoma; hepatoma; bile duct carcinoma; choriocarcinoma; seminoma; embryonal carcinoma; Wilms' tumor; cervical cancer; uterine cancer; testicular tumor; lung carcinoma; small cell lung carcinoma; bladder carcinoma; epithelial carcinoma; glioma; astrocytoma; medulloblastoma; craniopharyngioma; ependymoma; pinealoma; hemangioblastoma; acoustic neuroma; oligodendroglioma; meningioma; melanoma; retinoblastoma; and leukemias. In a preferred embodiment, the cancer is an epithelian cancer, ideally selected from the group comprising: breast; colorectal; ovarian; and prostate, and/or their metastases.

"Lower alkyl" means an alkyl group, as defined below, but having from one to ten carbons, more preferable from one to six carbon atoms (eg. "C - C - alkyl") in its backbone structure. "Alkyl" refers to a group containing from 1 to 8 carbon atoms and may be straight chained or branched. An alkyl group is an optionally substituted straight, branched or cyclic saturated hydrocarbon group. When substituted, alkyl groups may be substituted with up to four substituent groups, at any available point of attachment. When the alkyl group is said to be substituted with an alkyl group, this is used interchangeably with "branched alkyl group". Exemplary unsubstituted such groups include methyl, ethyl, propyl, isopropyl, a-butyl, isobutyl, pentyl, hexyl, isohexyl, 4, 4-dimethylpentyl, octyl, 2,2,4-trimethylpentyl, nonyl, decyl, undecyl, dodecyl, and the like. Examplary substituents may include but are not limited to one or more of the following groups: halo (such as F, CI, Br, I), Haloalkyl (such as CCl₃ or CF₃), alkoxy, alkylthio, hydroxyl, carboxy (-COOH), alkyloxycarbonyl (-C(O)R), alkylcarbonyloxy (-OCOR), amino (-NH2), carbamoyl (-NHCOOR-or-OCONHR), urea (-NHCONHR-) or thiol (-SH). Alkyl groups as defined may also comprise one or more carbon double bonds or one or more carbon to carbon triple bonds.

"Lower alkoxy" refers to O-alkyl groups, wherein alkyl is as defined hereinabove. The alkoxy group is bonded to the core compound through the oxygen bridge. The alkoxy group may be straight-chained or branched; although the straight-chain is preferred. Examples include methoxy, ethyloxy, propoxy, butyloxy, t-butyloxy, i-propoxy, and the like. Preferred alkoxy groups contain 1-4 carbon atoms, especially preferred alkoxy groups contain 1-3 carbon atoms. The most preferred alkoxy group is methoxy.

The terms "alkyl", "cycloalkyl", "heterocycloalkyl", "cycloalkylalkyl", "aryl", "acyl", "aromatic polycycle", "heteroaryl", "arylalkyl", "heteroarylalkyl", "amino acyl", "non-aromatic polycycle", "mixed aryl and non-aryl polycycle", "polyheteroaryl", "non-aromatic polyheterocyclic", "mixed aryl and non-aryl polyheterocycles", "amino", and "sulphonyl" are defined in US6,552,065, Column 4, line 52 to Column 7, line 39.

"Halogen" means the non-metal elements of Group 17 of the periodic table, namely bromine, chlorine, fluorine, iodine and astatine.

"Unidentate ligand" means an organic ligand and includes ammonia, a primary amine, a secondary amine, a non-planar heterocyclic aliphatic amine or a heterocyclic aromatic amine (such as pyridine, imidazole, indazole, bipyridine, phenantholine and derivatives), a sulphur donor ligand such as dimethyl sulfoxide, a phosphorous donor ligand such as a phosphine, or an oxygen donor ligand such as water or hydroxyl or hydroxide or methoxy or acetate

"Salt" is a pharmaceutically acceptable salt and can include acid addition salts such as the hydrochlorides, hydrobromides, phosphates, sulphates, hydrogen sulphates, alkylsulphates, arylsulphonates, acetates, benzoates, citrates, maleates, fumarates, succinates, lactates, and tartrates; alkali metal cations such as Na, K, Li; alkali earth metal salts such as Mg or Ca; or organic amine salts. Exemplary organic amine salts are tromethamine (TRIS) salts and amino acid salts (e.g. histidine salts) of the compounds of the invention.

The invention provides methods of, and compositions for, treatment and prevention by administration to a subject in need of such treatment of a therapeutically or prophylactically effective amount of a metal complex of the invention. The subject may be an animal or a human, with or without an established disease.

"Treating" (or "treat") as used herein includes its generally accepted meaning which encompasses prohibiting, preventing, restraining, and slowing, stopping or reversing progression, severity, of a resultant symptom. As such, the methods of this invention encompass both therapeutic and prophylactic administration.

"Effective amount" refers to the amount or dose of the compound, upon single or multiple dose administration to the patient, which provides the desired effect in the patient under diagnosis or treatment. An effective amount can be readily determined by the attending diagnostician, as one skilled in the art, by the use of known techniques and by observing results obtained under analogous circumstances. In determining the effective amount or dose of compound administered, a number of factors are considered by the attending diagnostician, including, but not limited to: the species of mammal; its size, age, and general health; the specific disease involved; the degree of or involvement or the severity of the disease; the response of the individual patient; the particular compound administered; the mode of administration; the bioavailabilty characteristics of the preparation administered; the dose regimen selected; the use of concomitant medication; and other relevant circumstances.

Various delivery systems are known and can be used to administer a compound or composition of the invention, e.g., encapsulation in liposomes, microparticles, microcapsules. Methods of introduction include but are not limited to intradermal, intramuscular, intraperitoneal, intravenous, subcutaneous, intranasal, epidural, and oral routes. The compounds or compositions may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local. In addition, it may be desirable to introduce the compounds or compositions of the invention into the circulation system by any suitable route. Pulmonary administration can also be employed, e.g., by use of an inhaler or nebulizer, and formulation with an aerosolizing agent.

It may be desirable to administer the compounds or compositions of the invention locally to the area in need of treatment; this may be achieved, for example and not by way of limitation, by topical application, by injection, by means of a catheter, by means of a suppository, or by means of an implant, said implant being of a porous, non-porous, or gelatinous material, including membranes, such as sialastic membranes, or fibers.

Alternatively, the compounds can be delivered in a vesicle, in particular a liposome (see Langer, Science 249:1527-1533 (1990); Treat et al., in Liposomes in the Therapy of Infectious Disease and Cancer, Lopez-Berestein and Fidler (eds.), Liss, New York, pp. 353-365 (1989); Lopez-Berestein, ibid., pp. 317-327).

In yet another embodiment, the compounds or compositions of the invention can be delivered in a controlled release system. In one embodiment, a pump may be used (see Langer, supra; Sefton, CRC Crit. Ref. Biomed., Eng. 14:201 (1987); Buchwald et al., Surgery 88:75 (1980); Saudek et al., N. Engl. J. Med. 321:574 (1989)). In another embodiment, polymeric materials can be used (see Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York(1984); Ranger and Peppas, J. Macromol. Sci. Rev. Macromol. Chem. 23:61 (1983); see also Levy et al., Science 228:190 (1985); During et al., Ann. Neurol. 25:351 (1989); Howard et al., J. Neurosurg. 71:105 (1989)). In yet another embodiment, a controlled release system can be placed in proximity of the therapeutic target, thus requiring only a fraction of the systemic dose (see, e.g., Goodson, in Medical Applications of Controlled Release, supra, vol. 2, pp. 115-138 (1984)). Other controlled release systems are discussed in the review by Langer (Science 249:1527-1533 (1990)).

The present invention also provides pharmaceutical compositions. Such compositions comprise a therapeutically effective amount of a metal complex of the invention, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals and more particularly in humans. The term "carrier" refers to a diluent, adjuvant, excipient, or vehicle with which the compound or pro-drug of the invention is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol and water.

The composition, if desired, can also contain minor amounts of wetting or emulsifying agents, or pH buffering agents. These compositions can take the form of solutions, suspensions, emulsion, tablets, pills, capsules, powders, sustained-release formulations and the like.

The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a therapeutically effective amount of the compound or pro-drug of the invention, preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to, ease pain at the, site of the injection. Generally, the ingredients are supplied either separately or mixed together in unit dosage form, for example, as a dry lyophilized powder or water free concentrate in a hermetically sealed container such as an ampoule or sachette indicating the quantity of active agent..

In the case of cancer, the amount of the therapeutic of the invention which will be effective in the treatment or prevention of cancer will depend on the type, stage and locus of the cancer, and, in cases where the subject does not have an established cancer, will depend on various other factors including the age, sex, weight, and clinical history of the subject. The amount of therapeutic may be determined by standard clinical techniques. In addition, in vivo and/or in vitro assays may optionally be employed to help predict optimal dosage ranges. The precise dose to be employed in the formulation will also depend on the route of administration, and the seriousness of the cancer, and should be decided according to the judgment of the practitioner and each patient's circumstances. Routes of administration of a therapeutic include, but are not limited to, intramuscularly, subcutaneously or intravenously. Effective doses may be extrapolated from dose-response curves derived from in vitro or animal model test systems.

The invention also provides a pharmaceutical pack or kit comprising one or more containers filled with one or more of the ingredients of the compositions of the invention.

### Experimental

### Synthesis of the Cu(II) complexes 1-6.

**Materials.** Chemicals, CT-DNA and duplex polymers were purchased from Sigma Aldrich (Ireland) and used without further purification. Supercoiled plasmid DNA (pUC18) was supplied by Roche Diagnostics Ltd (UK).

**Infrared Spectroscopy.** Solid-state IR were recorded in the region 4000 - 400 cm⁻¹ on a Nicolet FT-IR 5DXB infrared spectrometer with solid samples firstly being finely ground then mixed with KBr. Pellets consisted of 5 mg of analyte per 195 mg of KBr.

**Raman Spectroscopy.** Raman spectra were recorded from saturated potassium bromide pellets on an Instruments S.A. Labram 1B spectrometer with a confocal Raman imaging microscope system equipped with an Argon ion (514.5 nm, 50 mW) laser source. The light was imaged to a diffraction limited spot via the objective of an Olympus BX40 microscope and spectra were recorded over the range of 4000-150 cm⁻¹. The accumulation time was optimized for intensity; typically ten accumulations were recorded for each spectral window.

**Magnetic Susceptibility.** Measurements were made using a Johnson Matthey Magnetic Susceptibility balance. [HgCo(SCN)₄] was used as a reference.

**UV-vis Spectroscopy.** Samples were recorded in both solution and solid-state spectra using a Varian Cary 50 Scan single beam spectrophotometer which covered the range 800-190 nm using a Xenon lamp as light source. Solid-state samples were generated by finely grinding samples of each complex then drop-casting from a chloroform suspension onto a glass substrate. Solution-state spectra were recorded at 4.0 m*M* in DMSO.

**Conductivity.** Molar conductivity measurements were made at 25°C using an Orion 150 Aplus conductivity meter at 4.0 m*M* in DMSO.

**Elemental Analysis.** Microanalytical data for the complexes were reported by the Microanalytical Laboratory, University College Dublin, Ireland. Samples **1-6** were difficult to balance in terms of % hydrogen. The complexes all lost weight due to lattice molecules of water and ethanol being evaporated. We can account for water and ethanol molecules in the lattice from the IR and raman spectra of **1-6,** but some disparity does exists, in terms of molecules of hydration, between the X-ray and microanalytical results.

### Synthesis.

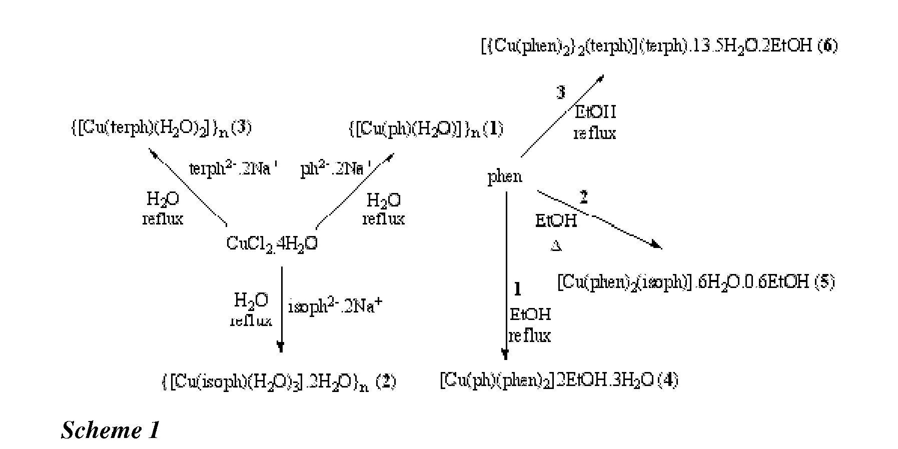

Cu(II) complexes **1-6** were synthesised according to *Scheme 1* above.
Complexes {[Cu(ph)(H₂O)]}ₙ (**1**), {[Cu(isoph)(H₂O)₃]2H₂O}ₙ (**2**) and {[Cu(tereph)(H₂O)₃]2H₂O}ₙ (**3**) were synthesised according to the following general procedure; to a hot solution of the relevant phthalic acid (7.5 g, 45 mmol) and sodium hydroxide (3.16 g, 80 mmol) in water (150 cm³) was added copper(II) chloride dihydrate (3.0 g, 22.31 mmol) with resultant blue solution being refluxed for 3 hr. The blue powder which deposited was filtered, washed with a small volume of ethanol and air-dried.
{[Cu(ph)(H₂O)]}*ₙ* (**1**); Yield: 4.62 g (81.33 %). % Calc: C, 40.60; H, 2.13 % Found: C, 40.49; H 2.43. IR (KBr): 456, 502, 574, 600, 658, 704, 717, 769, 798, 823, 834, 863, 887, 970, 999, 1034, 1086, 1144, 1160, 1262, 1298, 1375, 1444, 1486, 1571, 1592, 1613, 1638, 1855, 1889, 1933, 1964, 1995, 2134, 2235, 2531, 2741, 3064, 3461, 3512.95 cm⁻¹. Raman (KBr): 181, 235, 278, 317, 397, 463, 562, 598, 657, 721, 765, 798, 835, 1037, 1084, 1160, 1298, 1298, 1367, 1408, 1445, 1491, 1588, 1611, 3067, 3462 cm⁻¹. Solubility: DMSO/DMF. µ_{eff} = 1.47 B.M. UV-Vis (Solid-State): λ*_{d-d}* = 608 nm, UV-Vis (DMSO): *λ_{d-d}* = 608 nm, ε = 27 dm³ mol⁻¹ cm⁻¹, A*M* (DMSO): 27.30 S cm² mol⁻¹, {[Cu(isoph)(H₂O)₃]2H₂O}ₙ (2); Yield: 13.3 g (93.86 %). % Calc: C, 30.24; H, 4.44 % Found: C, 30.56; H 2.18. IR (KBr): 449, 477, 554, 660, 735, 800, 821, 928, 1002, 1077, 1102, 1162, 1177, 1273, 1313, 1327, 1385, 1444, 1480, 1565, 1612, 1685, 2562, 2674, 3396, 3591 cm⁻¹ Raman (KBr): 200, 463, 578, 659, 697, 726, 801, 825, 1004, 1181, 1293, 1409, 1457, 1573, 1611, 3075 cm⁻¹. Solubility: DMSO/DMF. µ_{eff} = 1.87 B.M. UV-Vis (Solid-State): λ*_{d-d}* = 671 nm, UV-Vis (DMSO): *λ_{d-d}* = 725 nm, ε = 145 dm³ mol⁻¹ cm⁻¹, A*M* (DMSO): 16.82 S cm² mol⁻¹, {[Cu(tereph)(H₂O)₅]}*ₙ* (3); Yield: 9.76 g (93.86 %). % Calc: C, 30.24; H, 4.44 % Found: C, 30.56; H 2.18. IR (KBr): 449, 477, 554, 660, 735, 800, 821, 928, 1002, 1077, 1102, 1162, 1177, 1273, 1313, 1327, 1385, 1444, 1480, 1565, 1612, 1685, 2562, 2674, 3396, 3591 cm⁻¹. Raman (KBr): 200, 463, 578, 659, 697, 726, 801, 825, 1004, 1181, 1293, 1409, 1457, 1573, 1611, 3075 cm⁻¹. Solubility: forms only suspensions in DMSO/DMF. µ_{eff} = 1.80 B.M. UV-Vis (Solid-State): *λ_{d-d}* = 636 nm.
Complexes [Cu(ph)(phen)₂]8H₂O (**4**), [Cu(isoph)(phen)₂]₅H₂O (**5**) and [{Cu{(phen)₂}₂(terph)](terph)10H₂O.3EtOH (**6**) were synthesised according to the general procedure; solutions of **1, 2** or **3** (2.0 mmol) in ethanol (50 cm³) were treated with 1,10-phenanthroline (0.73 g, 4.0 mmol) with resultant solutions being refluxed for 3 hr. In the synthesis of **4** and **6** blue powders deposited which were filtered and the resulting filtrates were allowed to stand for 1 week and 1 month, respectively, resulting in the formation of the corresponding crystals (which contain slight differences in molecules of hydration compared to their powders) suitable for X-ray crystallography. In the synthesis of **5**, a clear blue solution resulted, allowed to stand for 1 week resulting in the formation of crystals suitable for X-ray crystallography. [Cu(ph)(phen)₂]8H₂O (**4**); Yield: 0.40 g (27.99 %), % Calc: C, 52.49; H, 4.69; N, 7.65, % Found: C, 52.45; H, 2.87; N, 7.50 IR (KBr): 410, 622, 694, 738, 778, 853, 987, 1090, 1137, 1216, 1345, 1421, 1504, 1504, 1561, 1586, 1615, 3058, 3765 cm⁻¹. Raman (KBr): 246, 410, 551, 709, 1053, 1097, 1276, 1295, 1405, 1447, 1503, 1563, 1588, 3065 cm⁻¹. Solubility: forms only suspensions in DMSO/DMF. µ_{eff} = 1.80 B.M. UV-Vis (Solid-State): λ*_{d-d}* = 682 nm, [Cu(isoph)(phen)₂]5H₂O (5); Yield: 0.07 g (5.47 %), % Calc: C, 56.68; H, 4.46; N, 8.26, % Found: C, 56.80; H, 3.33; N, 8.17 IR (KBr): 431, 647, 723, 748, 816, 853, 902, 942, 996, 1067, 1103, 1143, 1223, 1263, 1308, 1314, 1356, 1379, 1426, 1450, 1472, 1493, 1518, 1577, 1604, 3060, 3405 cm⁻¹. Raman (KBr): 190, 309, 429, 560, 739, 817, 1002, 1054, 1255, 1305, 1343, 1370, 1430, 1455, 1518, 1585, 1606, 1626, 3078 cm⁻¹. Solubility: DMSO/DMF. µ_{eff} = 2.02 B.M., UV-Vis (Solid-State): λ*_{d-d}* = 638 nm, UV-Vis (DMSO): *λ_{d-d}* = 675 nm, ε = 35 dm³ mol⁻¹ cm⁻¹, AM (DMSO): 2.25 S cm² mol⁻¹, [{Cu{(phen)₂}₂(terph)](terph)10H₂O.3EtOH (**6**) Yield: 0.78 g (52.19 %). % Calc: C, 56.26; H, 4.73; N, 7.50, % Found: C, 56.11; H, 3.19; N, 7.30. IR (KBr): 429, 452, 507, 546, 593, 647, 722, 743, 783, 825, 850, 868, 887, 1013, 1031, 1050, 1089, 1105, 1144, 1197, 1224, 1309, 1344, 1374, 1385, 1427, 1498, 1517, 1569, 1602, 1625, 1693, 3057, 3388cm⁻¹. Raman (KBr): 274, 312, 436, 567, 611, 744, 865, 1062, 1137, 1256, 1319, 1436, 1523, 1614, 3082 cm⁻¹. Solubility: forms only suspensions in DMSO/DMF. µ_{eff} = 1.97 B.M. UV-Vis (Solid-State): *λ_{d-d}* = 575 nm, 700 nm (sh).

### X-ray crystallographic data for 4-6.

Complexes **4-6** were characterised by single X-ray diffraction as [Cu(ph)(phen)₂]3H₂O.2EtOH, [Cu(isoph)(phen)₂]6H₂O.EtOH and [{Cu{(phen)₂}₂(terph)](terph)13.5H₂O.2EtOH respectively. For complexes **4** and **5** single crystal X-ray diffraction data were collected on a Bruker X8 APEX2 CCD diffractometer (Bruker 2004, APEX2 Version 2.1 Bruker AXS Inc., Madison, Wisconsin, USA) using the APEX2 suite of programs and further analysed using PLATON. Water molecule hydrogen atoms were located via the program CALC-OH. Data for complex **6** were collected at 153(2) K using a Siemens P4 diffractometer and corrected for Lorentz, polarisation and absorption effects. The structure was solved by direct methods and refined by full matrix least-squares on F². The asymmetric unit contains half a cation, half a terephthalate dianion, six well-ordered water molecules and a disordered solvate region. The disorder was modelled as containing an ethanol molecule distributed over three sites (50:25:25) and a further 75% occupancy water molecule, also disordered over two sites (50:25). All the non-hydrogen atoms were refined with anisotropic atomic displacement parameters, except for the disordered solvate at 25% occupancy. Hydrogen atoms bonded to full-occupancy carbon atoms were inserted at calculated positions, those bonded to oxygen atoms were located from difference Fourier maps and refined under restraints, those 25% occupancy oxygen atoms were not included. All programs used in the structure solutions and refinements are contained in the SHELXTL package.

### Cytotoxicity Assays (For Tested Complexes 1-6).

**Human colon cancer cell line, HT29** (passage 17 to 30, ATCC, USA), was grown in McCoys's 5a medium supplemented with 2mM L-glutamine and fetal bovine serum (FBS) 10% and containing 100U/ ml penicillin and 100µg / ml streptomycin at 37°C in a humidified atmosphere with 5% CO₂
**Human Breast Cancer cell line, MCF-7** (passage 16 to 26, ATCC, USA) was grown in Eagle's minimum essential medium containing 2mM L-glutamine, 100U/ ml penicillin,100µg/ ml streptomycin, FBS (10%), 1mM sodium pyruvate and 1% (v/v) non essential amino acids at 37°C in a humidified atmosphere with 5% CO₂
**Human prostate cancer cell line, DU145** (passage 12 to 29, ATCC, USA) was grown in Eagle's minimum essential medium containing 2mM L-glutamine, 100U/ ml penicillin, 100µg/ ml streptomycin, FBS (10%), 1mM sodium pyruvate and 1% (v/v) non essential amino acids at 37°C in a humidified atmosphere with 5% CO₂.
All drugs were tested by MTT assay following 24-hour exposure of cells to each compound. Each value represents the mean IC₅₀ of three independent experiments +/- standard error.

**MTT Assay.** This method is based on the reduction of the tetrazolium salt, methylthiazolyldiphenyl-tetrazolium bromide (MTT) into a crystalline blue formazan product by the cellular oxidoreductases of viable cells. The resultant formazan crystal formation is proportional to the number of viable cells. Cells were seeded at 4x 10⁵ cells/ml in 96-well plates and incubated at 37°C in 5% CO₂ for 24 hours. Cells were treated with a four log range of concentration of the test compounds in triplicate from 0.1 to 500 µM or with a solvent control (0.5% DMSO) in complete medium. Following 24 h incubation, cells were incubated with 20 µl of MTT (5 mg/ml) in 0.1 M PBS, pH 7.4 at 37 °C in a humid atmosphere with 5% CO₂ for 4 h. Media was then gently aspirated from test cultures and 100 µL of dimethyl sulphoxide (DMSO) was added to all wells. The plates were then shaken for 2 min and the absorbance was read at 550 nm in a Varioscan plate reader. The IC₅₀ was defined as the concentration of test compound required to reduce the absorbance of the MTT-formazan crystals by 50%, indicating 50% cell deactivation.

### Cytotoxicity Assays (Complex 7).

### Cell Culture.

For cytotoxicity evaluation HT29, SW480 and SW620 cell lines were employed; all the cell lines were grown in RPMI 1640 media (Fischer). All media were supplemented with 10% foetal bovine serum (FBS) and 45 IU/ml penicillin and 45 g/ml streptomycin and cells were maintained at 37 °C in a 5% CO₂ humidified incubator.

### Test Sample Preparation.

For sterilisation samples were filtered with a 0.2 micron cellulose acetate filter.
For cytotoxicological evaluation all samples were prepared fresh on the day of exposure. A stock concentration of test compound was prepared in the exposure media (10% DMEM F-12) necessary for the cell line under test. This sample was vortexed to ensure a uniform dispersion of the compound under test.

### Cytotoxicity analysis using the MTT assay

For cytotoxicity assays cells were seeded in 96-well microplates (Nunc, Denmark) at a density of 1 x 10⁵ cells/ml for the 24 hr test, 5 x10⁴ cells/ml for the 48 hr test and 3 x 10⁴ cells/ml for the 96 hr test. These densities were found to be optimal to achieve the desired confluence at the end of the exposure period. After 24 h of cell attachment, plates were washed with 100 µl/well phosphate buffered saline (PBS) and the cells were treated with increasing concentrations of each compound for 24 h. Six replicate wells were used for each control and test concentration per microplate. Following the desired time of compound exposure, control medium or test exposures were removed, the cells were rinsed with PBS and 100 µl of fresh medium (without FBS or supplements) was added to each well. 10 µl of MTT (5 mg/ml) prepared in PBS was then added to each well and the plates were incubated for 3 h at 37°C in a 5% CO₂ humidified incubator. After this incubation period the medium was discarded, the cells were washed with 100 µl of PBS and 100 µl of DMSO was added to each well to extract the dye. The plate was shaken at 240 rpm for 10 min and the absorbance was measured at 570 nm.

### Data Analysis.

At least three independent experiments were conducted in triplicate for each cell line and toxicity endpoint. Test results for each test were expressed as percentage of the unexposed control ± standard deviation (SD). Control values were set as 100%. Differences between samples and the control were evaluated using the statistical analysis package SPSS 14.0. Statistically significant differences were set at p≤0.05. One-way analysis of variances (ANOVA) followed by Dunnett's multiple comparison tests were carried out for normally distributed samples with homogeneous variances. Cytotoxicity data (where appropriate) was fitted to a sigmoidal curve and a four parameter logistic model used to calculate the LD50 values for each test sample at various time points, which was the concentration of nanomaterial which caused a 50% cellular lethality in comparison to untreated controls. The LD50 values are reported ± 95 % Confidence Intervals (± 95% CI).

### Data acquisition.

For cytotoxicity evaluation fluorescence measurements were all quantified using a microplate reader (TECAN GENios, Grödig, Austria).

### Competitive ethidium displacement and quenching experiments.

**Competitive Ethidium Displacement.** A working solution containing 1 µ*M* CT-DNA (ε₂₆₀ = 12,824 *M*(bp)⁻¹ cm⁻¹) along with 1.26 µ*M* ethidium bromide (EtBr) at neutral pH in TES buffer (10 m*M* TES, 0.1 m*M* Na₂EDTA, pH = 7.0) was prepared. Complexes **4-6**, actinomycin D, pentamidine isethionate were prepared at 2.0 m*M* in DMSO. Two millilitres of DNA-Et solution were placed in a 10-mm quartz cuvet (3 mL) and positioned in a temperature controlled (20°C) spectrofluorometer (Perkin Elmer LS55B Luminescence Spectrometer). Excitation and emission wavelengths were set to 546 and 595 nm, respectively. After thermal equilibrium was established the emission and excitation slits were modified to give a fluorescence reading of 50 arbitrary units with measurements being recorded over a 20-s interval. An aliquot of complex or drug solution was taken (0.5 - 10 µL), was added to the cuvette and after equilibration the fluorescence reading was recorded. Repeated aliquots were added until the fluorescence was 20-40% of the initial control. Triplicate titrations were preformed and the apparent binding constants were calculated using *K*app *= Kₑ* x 1.26/C₅₀ where *Kₑ* = 9.5 x 10⁶ *M*(bp)⁻¹.

**DNA-Ethidium Fluorescence Quenching.** Working solutions containing 20 µ*M* poly[d(AT)]₂ (ε₂₆₀ = 13,100 *M*(bp)⁻¹ cm⁻¹), poly[d(G-C)]₂ (ε₂₆₀ = 16,800 *M*(bp)⁻¹ cm⁻¹) and CT-DNAp (ε₂₆₀ = 12,824 *M*(bp)⁻¹ cm⁻¹) along with 2.0 µ*M* Et in NaOAc buffer (2 m*M* NaOAc, 9.3 m*M* NaCl, 0.1 m*M* Na₂EDTA, pH = 5.0) were prepared. Subsequent experimental steps are identical to the displacement experiment with the exception that a fluorescence reading of 200 arbitrary units with measurements being recorded over a 20-s interval was used. From a plot of the fluorescence vs added drug concentration, the *Q* values is given by the concentration required to effect 50% removal of the initial fluorescence.

### Time-course experiments with DNA cleavage.

**Nuclease Activity.** Reactions were carried out in a total volume of 20 µL in 0.1 *M* cacodylate buffer (pH = 6.0) with either 0.5, 2.5, 10 µL of 40 µ*M* complex or 2, 5, 10 µL of 200 µ*M* of complex, which were initially prepared in DMF, then diluted in cacodylate buffer (in which they dissolved), with 1 µL of 0.25 µg/µL pUC18 in cacodylate buffer. The resulting mixtures contained 1, 5, 10, 20, 50 and 100 µ*M* of complex and 12 ng of pUC18. Samples were incubated for 5 hr at 37°C. Quench buffer (3 µL; 0.25% bromophenolblue, 0.25% xylene cyanole and 30% glycerol) was then added and samples were loaded onto agarose gel (0.8%) containing 1.5 µL/100 mL of solution of GelRed^{™} (10,000X). Electrophoresis was completed at 80 V for 2 hr in 1XTAE buffer.

### Results (Complexes 1-6)

The cytotoxicity of **1-6** along with their free ligands and the clinical antitumor agents cisplatin and mitoxantrone were studied using a standard MTT assay against four Human-derived tumor lines (***Table 1***). It should be noted that ovarian adenocarinoma (SK-OV-3) cells are resistant to tumor necrosis factor and to several cytotoxic drugs including diphtheria toxin, cisplatin and adriamycin. In addition colorectal adenocarcinoma (HT29, grade II) malignancies have proven difficult to treat with cisplatin. Cu(II) bis-phen complexes **4-6** display rapid, low micomolar toxicities against all tumor lines. Results for this group are comparable to mitoxantrone against HT29, however significant improvements were noted in both DU145 and SK-OV- 3 lines. Complex **6** showed the best broad-spectrum activity and was the most active complex against breast adenocarcinoma (MCF-7). It should be noted that **6** is dinuclear and so contains two active copper units, which may explain this heightened potency. It is also significant that the free ligands are essentially inactive against all tumor lines.

**Table 1 IC₅₀ values (µM) obtained from the complexes, free ligands and clinically used antitumor agents Cisplatin and mitoxantrone against breast cancer (MCF-7), prostate cancer (DU145), ovarian cancer (SK-OV-3) and colon cancer cell line (HT29) over a period of 24 hours.**

| | Activity IC₅₀ (µM) 24 hr | | | |
|---|---|---|---|---|
| | MCF-7 | DU145 | SK-OV-3 | HT29 |
| phen | 337.7 ± 64.7 | 272.5 ± 8.0 | 286 ± 28.5 | 376.8 ±9.2 |
| phH₂ | >500 | >500 | >500 | >500 |
| isophH₂ | >500 | >500 | >500 | >500 |
| terphH₂ | >500 | >500 | >500 | >500 |
| **1** | >500 | >500 | >500 | >500 |
| **2** | 401.2 ± 54.1 | 422.5 ± 22.4 | 436.3 ± 29.4 | >500 |
| **3** | 414.3 ± 26.0 | >500 | >500 | >500 |
| **4** | 44.9 ± 7.0 | 11.6 ± 4.5 | 6.7 ± 0.4 | 6.0 ± 0.4 |
| **5** | 41.2 ± 1.4 | 10.6 ± 2.2 | 6.6 ± 0.4 | 5.8 ± 0.2 |
| **6** | 7.9 ± 0.4 | 5.7 ± 0.2 | 6.7 ± 0.4 | 5.4 ± 0.3 |
| mitoxantrone | 7.5 ± 0.2 | 27.3 ± 5.6 | 54.5 ± 2.6 | 8 ± 0.6 |
| Cisplatin | 90.8 ± 3.5 | >500 | >500 | 348.6 ± 80.6 |

**Table 2: Apparent binding constants (Kₐₚₚ) of 4-6 along with actinomycin D and pentamidine. Assay conditions; final volume 2 mL, 1.2 µM EtBr, 1 µM CT-DNAp, 10 mM TES, 0.1 mM Na₂EDTA, pH 7.0**

| Drug | *C*₅₀^{*}(µ*M*) | *K*ₐₚₚ^{**} |
|---|---|---|
| actinomycin D | 12.35 | 9.69 x 10⁵ |
| pentamidine | 19.72 | 6.07x10⁵ |
| **4** | 99.06 | 1.21x10⁵ |
| **5** | 99.79 | 1.20x10⁵ |
| **6** | 39.36 | 3.04x10⁵ |

| | | |
|---|---|---|
| ^{*}C₅₀ = concentration required to reduce fluorescence by 50% ^{**}Kₐₚₚ = *Kₑ* x 1.26/C₅₀ where *Kₑ* = 9.5 x 10⁶ *M*(bp)⁻¹ | | |

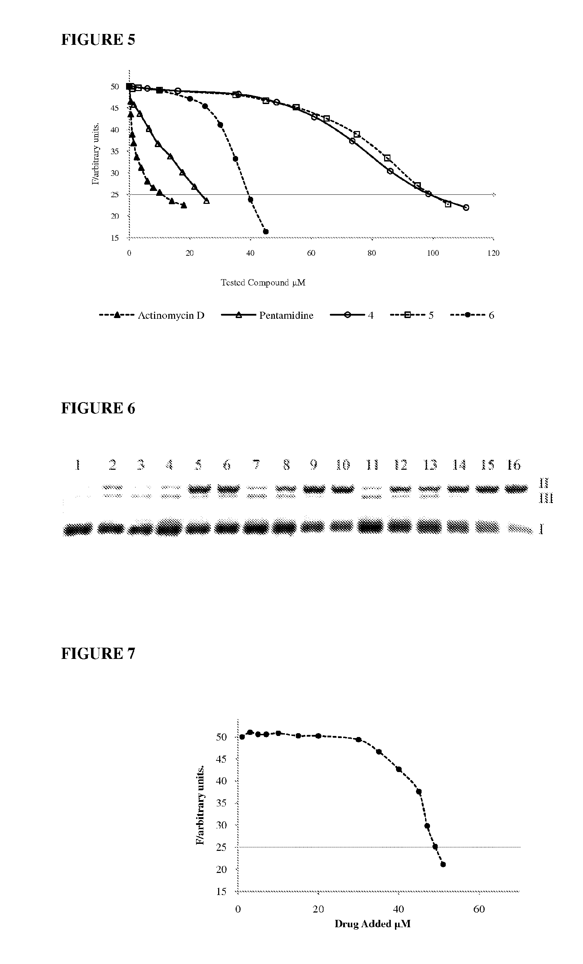

As the complexes exhibited poor molar extinction coefficients (ε) or were only part soluble in DMF/DMSO an indirect fluorometric method of examining their DNA binding capabilities was chosen. Competitive ethidium bromide (EtBr) displacement experiments were conducted with **1-6** along with the known intercalator, actinomycin D, and the minor groove binder, pentamidine, using calf thymus DNA (CT-DNA) (***Figure 5******, Table 2***). Et-bound DNA is highly fluorogenic and in the presence of excess Et, binding regions within the DNA polymer become saturated. Thus, during competitive displacement, an exogenous reagent must 'compete' at the binding site with Et resulting in a sequential reduction in fluorescence. Complexes **1-3** are devoid of binding ability but phen containing complexes **4-6** do interact DNA. Actinomycin D and pentamidine are highly efficient in the displacement of Et bound DNA and, as expected, their apparent binding constants (*K*ₐₚₚ) are high. Comparably complexes **4-6** have lower, but notable, *K*ₐₚₚ constants with dinuclear **6** showing superiority over mononuclear **4** and **5.**

**Table 3: Q values of poly[d(A-T)]₂ and poly[d(G-C)]₂ for actinomycin D, pentamidine and complex 4-6. Assay conditions; final volume 2 mL, 2.0 µM EtBr, 20 µM DNAp, 2 mM NaOAc buffer, 9.3 mM NaCl, 0.1 mM Na₂EDTA, pH 5.0**

| Drug | *Q*^{†} poly[d(A-T)₂] (µ*M*) | *Q*^{†} poly[d(G-C)₂] (µ*M*) |
|---|---|---|
| actinomycin D | 314.00 | 4.25 |
| pentamidine | 44.89 | 203.40 |
| **4** | 26.22 | 69.81 |
| **5** | 20.87 | 37.32 |
| **6** | 8.55 | 10.28 |

| | | |
|---|---|---|
| ^{†}*Q* = equivalent concentration required to reduce fluorescence by 50% | | |

In an effort to elucidate the binding mode of complexes **4-6,** fluorescence quenching of duplex adenine-thymine (A-T) and guanine-cytosine (G-C) polymers were conducted. Under conditions of limited Et bound to an excess of DNA, exogenous intercalating agents demonstrate high affinities toward G-C base pairs while minor groove binding species preference A-T base paired regions. ***Table 3*** reports *Q* values of actinomycin D (intercalator) and pentamidine (groove-binder) along with complexes **4-6.** Noted is the specificity to which actinomycin and pentamidine quench fluorescence within specific oligopolymers; indicative of classical intercalator and groove-binding behaviour. Complexes **4-6** display greater affinities toward poly[d(A-T)]₂ than pentamidine but low *Q* values are also evident within poly[d(G-C)]₂; factors which may indicate flexible or multimodal binding.

Relaxation of supercoiled (SC) pUC18 DNA (Form I) into open circular (OC, Form II) and linear (LC, Form III) conformations was used to quantify the relative cleavage efficiency of complexes **4-6.** SC DNA was exposed to **4-6** over a general concentration range of 1-100 µ*M* for 5 hours in the absence of added H₂O₂ or reductant (***Figure 6***). All complexes show concentration-dependant relaxation of SC (Form I) DNA to OC (Form II) while some LC (Form III) activity is evident but only at lower concentrations. The overall trend in 'self-activating' nuclease activity is **6>>4> 5.** Indeed complex **6** displayed some activity at lower concentrations of 1-5 µ*M* (lanes 11 & 12) with almost complete depletion of the parent SC bands in both **6** (I→II) being witnessed at higher concentrations (lanes 15 & 16).

### Results (Complex 7)

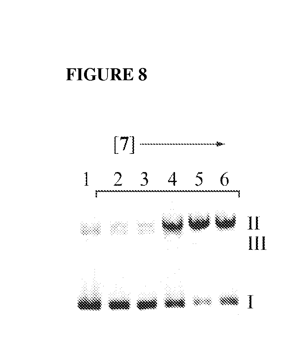

A dinuclear dicationic Cu(II) bis-phenanthroline complex containing a bridging suberate ligand is charge-balanced by two perchlorate anions in the crystal lattice. The complex [Cu₂(oda)(phen)₄](ClO₄)₂.2.67H₂O.C₂H₅OH (Complex **7)** was generated and characterised according to the published method by M. Devereux et. al, Polyhedron 1999, 18, 2141-2148. The complex exhibited rapid, low-micromolar chemotherapeutic potential, and was tested along with the free phen ligand and the clinically used antitumour agent cisplatin, using three (increasingly aggressive) colorectal cancer cell lines; HT29, SW480 (Dukes' B) and SW620 (Dukes' C) using a standard MTT assay (***Table 4***). The complex displayed an efficient ability to bind with calf thymus DNA (CT-DNA) in both an ethidium bromide displacement (***Figure** 7**,* ***Table* 5**) and a quenching assay (***Table 6***). The relaxation of supercoiled (SC) pUC18 DNA (Form I) into open circular (OC, Form II) and linear (LC, Form III) conformations was used to quantify the relative cleavage efficiency (nuclease activity) of the complex in the absence of added oxidant or reductant **(*****Figure* 8****).** Complex **7** was found to be highly efficient in the cleavage of plasmid DNA with noteable activity from 10 µ*M* onwards.

**Table 4: LD₅₀ values (µM) for complex 7, the free ligand phen and the antitumour agent cisplatin, against colon cancer lines HT29, SW480 and SW620 over a period of 24 hours**

| | Activity LD₅₀ (µ*M*) 24 hr | | |
|---|---|---|---|
| | HT29 | SW480 | SW620 |
| phen | >100 | >100 | >100 |
| cisplatin | >100 | >100 | >100 |
| **7** | 9.61 | 11.3 | 31 |

**Table 5: Apparent DNA binding constant (Kₐₚₚ) for complex 7. Assay conditions; final volume 2 mL, 1.2 µM EtBr, 1 µM CT-DNAp, 10 mM TES, 0.1 mM Na₂EDTA, pH 7.0**

| Drug | *C*₅₀^{*} (µ*M*) | *K*ₐₚₚ^{**} |
|---|---|---|
| **7** | 46.89 | 2.55x10⁵ |

| | | |
|---|---|---|
| ^{*}C₅₀ = concentration required to reduce fluorescence by 50% ^{**}*K*ₐₚₚ = *K*ₑ x 1.26/C₅₀ where K*ₑ* = 9.5 x 10⁶ *M*(bp)⁻¹ | | |

**Table 6: Q values of CT-DNAp for complex 7. Assay conditions; final volume 2 mL, 2.0 µM EtBr, 20 µM DNAp, 2 mM NaOAc buffer, 9.3 mM NaCl, 0.1 mM Na₂EDTA, pH 5.0**

| Drug | *Q*^{†} CT-DNA (µ*M*) |
|---|---|
| **7** | 22.64 |

| | |
|---|---|
| ^{†}*Q* = equivalent concentration required to reduce fluorescence by 50% | |

The invention is not limited to the embodiments hereinbefore described which may be varied in construction and detail without departing from the spirit of the invention.

### REFERENCES

1. D. S. Sigman, D. R. Graham, V. D'Aurora and A. M. Stern, J. Biol. Chem., 1979, 254, 12269-12272.
2. M. Pitie and G. Pratviel, Chem. Rev., 2010, 110, 1018-1059.
3. M. Pitie, C. J. Burrows and B. Meunier, Nucleic Acids Res., 2000, 28, 4856-4864.
4. E. Lamour, S. Routier, J.-L. Bernier, J.-P. Catteau, C. Bailly and H. Vezin, J. Am. Chem. Soc., 1999, 121, 1862-1869.
5. M. S. Melvin, J. T. Tomlinson, G. R. Saluta, G. L. Kucera, N. Lindquist and R. A. Manderville, J. Am. Chem. Soc., 2000, 122, 6333-6334.
6. M. S. Melvin, K. E. Wooton, C. C. Rich, G. R. Saluta, G. L. Kucera, N. Lindquist and R. A. Manderville, J. Inorg. Biochem., 2001, 87, 129-135.
7. P. U. Maheswari, M. v. d. Ster, S. Smulders, S. Barends, G. P. v. Wezel, C. Massera, S. Roy, H. d. Dulk, P. Gamez and J. Reedijk, Inorganic Chemistry, 2008, 47, 3719-3727.
8. L. Li, K. D. Karlin and S. E. Rokita, J. Am. Chem. Soc., 2004, 127, 520-521.
9. S. van der Steen, P. de Hoog, K. van der Schilden, P. Gamez, M. Piti, R. Kiss and J. Reedijk, Chem. Commun., 2010, 46, 3568-3570.
10. W. A. E. McBryde, D. A. Brisbin and H. Irving, J. Chem. Soc., 1962, 5245-5253.
11. H. I. a. D. H. Mellor, J. Chem. Soc., 1962, 5237-5245.
12. A. D. Randford and P. J. Sadler, Dalton Trans., 1993, 3393-3399.
13. M. Geraghty, V. Sheridan, M. McCann, M. Devereux and V. McKee, Polyhedron, 1999, 18, 2931-2939.
14. M. A. Zoroddu, S. Zanetti, R. Pogni and R. Basosi, J. Inorg. Biochem., 1996, 63, 291-300.
15. Y. Sun, FEBS Lett., 1997, 408, 16-20.
16. Y. Sun, J. Bian, Y. Wang and C. Jacobs, Oncogene, 1997, 14, 385-393.
17. D. Sun, R. Cao, Y. Liang, M. Hong, W. Su and J. Weng, Acta Cryst., 2000, 56C, e240-e241.

## Claims

1. A metal complex for use in treating cancer, the metal complex having a general structure I:
**I** [Y₂-X-Y₁]ⁿ⁺ • B
in which:
- X is an optionally substituted, branched or unbranched, aliphatic dicarboxylic acid, or a monocyclic or polycyclic aromatic, non-aromatic, or heterocyclic, dicarboxylic acid;
- Y₁ is Cu²⁺Phen)₂L₁ having the structure: 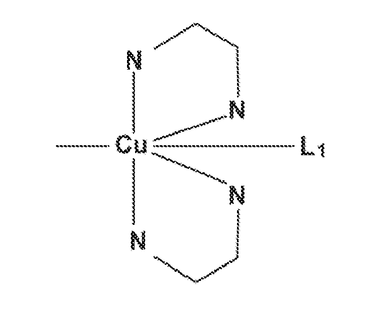 in which: 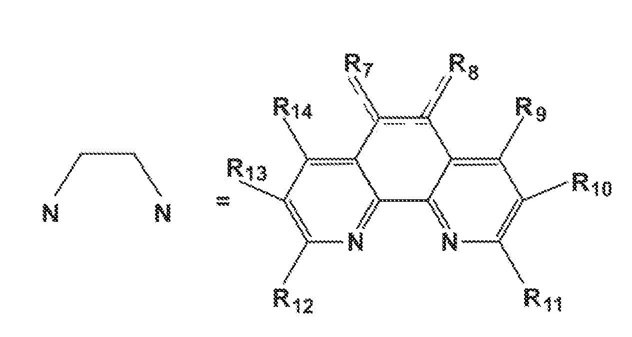 Cu²⁺ is coordinated to an oxygen atom of a carboxylate group of X;
R₇ to R₁₄ are each, independently, selected from oxygen, hydrogen, hydroxyl, a carboxyl, a substituted or unsubstituted, branched or unbranched alkyl, alkenyl, cycloalkyl, aryl, aryloxy, alkyloxy, arylalkyloxy, amino, alkylamino, hydroxylamino, dialkylamino, or alkoxy group, or pyridine, or are fused to form a monocyclic or polycyclic aromatic hydrocarbon or heterocycle;
L₁ is absent or is a unidentate ligand; and
a broken line designates a single or double bond,
- Y₂ is absent, or when present is Cu²⁺(Phen)₂L₁ in which the copper ion is coordinated to an oxygen atom of a carboxylate group of X;
- n⁺ is the charge on the complex ion or is absent; and
- B is a counterion or is absent.

2. Use as claimed in Claim 1 in which X is an optionally substituted, branched or unbranched, aliphatic dicarboxylic acid.

3. Use as claimed in Claim 2 in which the metal complex is a mono-nuclear Cu²⁺ complex having a formula: 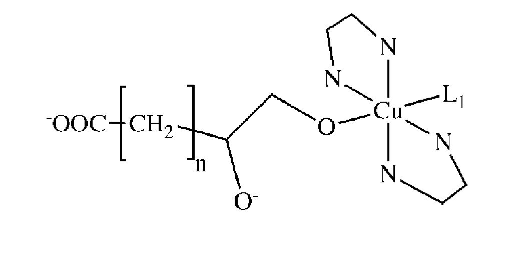 in which:
- n = 0 to 20.

4. Use as claimed in Claim 2 in which the metal complex is a di-nuclear Cu²⁺ complex having a formula:

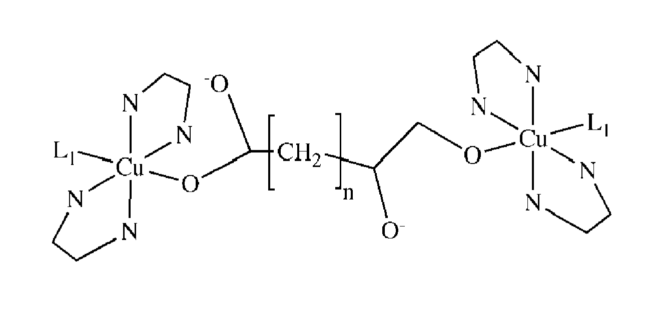

5. Use as claimed in Claim 1 in which X is an optionally substituted aromatic dicarboxylic acid.

6. Use as claimed in Claim 5 in which the metal complex has a general structure **II**: 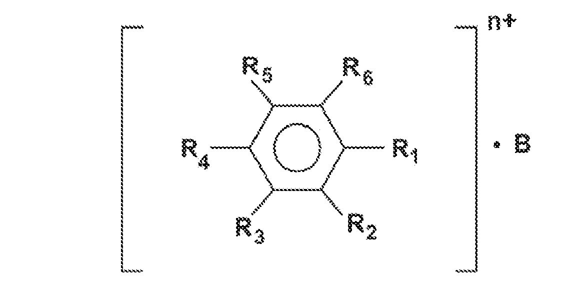 in which:
R₁ to R₆ are each, independently, selected from oxygen, hydrogen, hydroxyl, a carboxyl, a substituted or unsubstituted, branched or unbranched alkyl, alkenyl, cycloalkyl, aryl, aryloxy, alkyloxy, arylalkyloxy, amino, alkylamino, hydroxylamino, dialkylamino, or alkoxy group, or pyridine, or are fused to form a monocyclic or polycyclic aromatic hydrocarbon or heterocycle, or is a substituent
-Z-COO⁻-Cu²⁺(Phen)₂ having the formula: 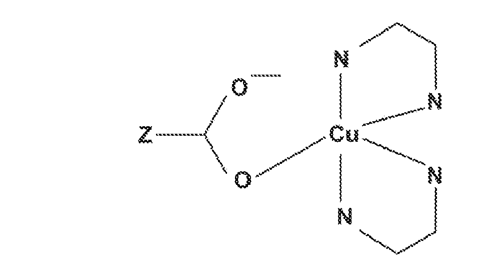 in which: 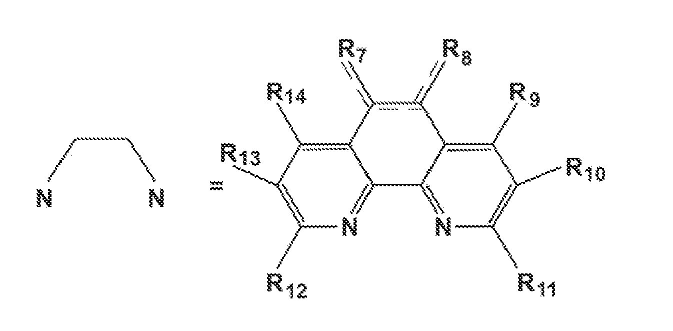 R₇ to R₁₄ are each, independently, selected from oxygen, hydrogen, hydroxyl, a carboxyl, a substituted or unsubstituted, branched or unbranched alkyl, alkenyl, cycloalkyl, aryl, aryloxy, alkyloxy, arylalkyloxy, amino, alkylamino, hydroxylamino, dialkylamino, or alkoxy group, or pyridine, or are fused to form a monocyclic or polycyclic aromatic hydrocarbon or heterocycle;
a broken line designates a single or double bond; and
Z is a spacer group or is absent,
- n+ is the charge on the complex ion, or is absent; and
B is a counterion or is absent,
wherein one or two of R₁ to R₆ is -Z-COO⁻-Cu²⁺(Phen)₂, and wherein when one of R₁ to R₆ is -Z-COO⁻-Cu²⁺(Phen)₂, another of R₁ to R₆ is a carboxylate substituent.

7. A method as claimed in Claim 5 or 5 in which the charge on the complex ion (n+) is 2+, and the counterion is phthalic acid, or a derivative thereof.

8. Use as claimed in any preceding Claim in which each Phen is 1,10 phenanthroline.

9. Use as claimed in any of Claims 6 to 8 in which the metal complex is a mono-nuclear Cu²⁺ complex in which one of R₁ to R₆ is -Z-COO⁻-Cu²⁺(Phen)₂, one of R₁ to R₆ is a carboxylate group, and the remainder of R₁ to R₆ are H.

10. Use as claimed in any of Claims 6 to 8 in which the metal complex is a dinuclear Cu^{II} complex in which two of R₁ to R₆ are -Z-COO⁻-Cu²⁺(Phen)₂ substituents, the remainder of R₁ to R₆ are H, and the counterion B is suitably a phthalic acid or phthalic acid derivative.

11. Use as claimed in any preceding Claim in which the metal complex contains a moiety which is capable of targeting a polynucleic acid sequence.

12. A metal complex having a general structure **II**: 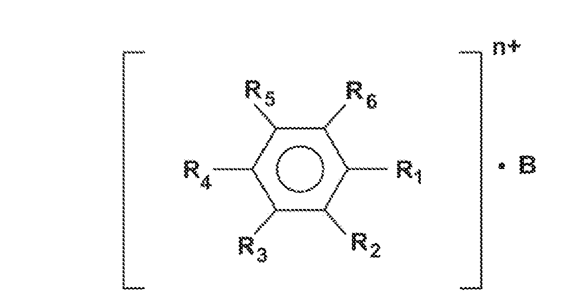 in which:
R₁ to R₆ are each, independently, selected from oxygen, hydrogen, hydroxyl, a carboxyl, a substituted or unsubstituted, branched or unbranched alkyl, alkenyl, cycloalkyl, aryl, aryloxy, alkyloxy, arylalkyloxy, amino, alkylamino, hydroxylamino, dialkylamino, or alkoxy group, or pyridine, or are fused to form a monocyclic or polycyclic aromatic hydrocarbon or heterocycle, or is a substituent
-Z-COO⁻-Cu²⁺(Phen)₂ having the formula: 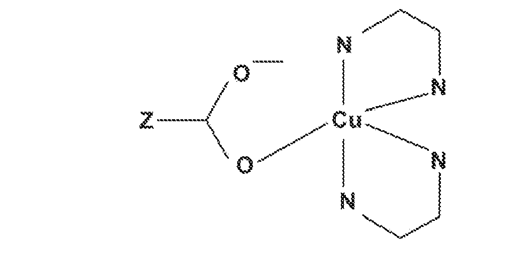 in which: 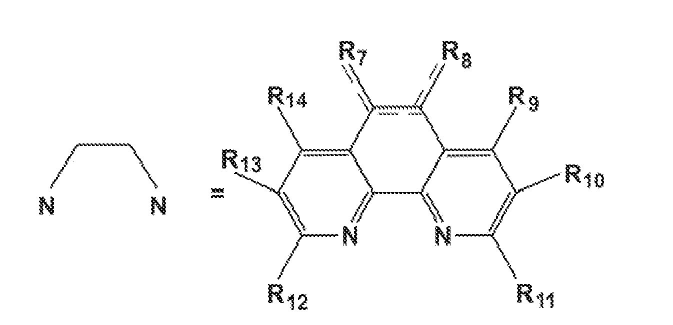 R₇ to R₁₄ are each, independently, selected from oxygen, hydrogen, hydroxyl, a carboxyl, a substituted or unsubstituted, branched or unbranched alkyl, alkenyl, cycloalkyl, aryl, aryloxy, alkyloxy, arylalkyloxy, amino, alkylamino, hydroxylamino, dialkylamino, or alkoxy group, or pyridine, or are fused to form a monocyclic or polycyclic aromatic hydrocarbon or heterocycle;
a broken line designates a single or double bond; and
Z is a spacer group or is absent,
- n+ is the charge on the complex ion, or is absent; and
B is a counterion or is absent,
wherein one or two of R₁ to R₆ is -Z-COO⁻-Cu²⁺(Phen)₂, and wherein when one of R₁ to R₆ is -Z-COO⁻-Cu²⁺(Phen)₂, another of R₁ to R₆ is a carboxylate substituent, with the proviso that the counterion B is not 2ClO₄⁻.

13. A metal complex as claimed in Claim 12 in which the charge (n+) on the complex ion is 2+, and the counterion is phthalic acid, or a derivative thereof.

14. A metal complex as claimed in Claim 12 or 13 and selected from:
(a) a mono-nuclear Cu²⁺ complex in which one of R₁ to R₆ is -Z-COO⁻-Cu²⁺(Phen)₂, one of R₁ to R₆ is a carboxylate group, and the remainder of R₁ to R₆ are H; or
(b) a dinuclear Cu^{II} complex in which two of R₁ to R₆ are -Z-COO⁻-Cu²⁺(Phen)₂ substituents, the remainder of R₁ to R₆ are typically H, and the counterion B is suitably a phthalic acid or phthalic acid derivative.

15. A metal complex as claimed in Claim 14 and selected from the group consisting of:
- R₁ is -Z-COO⁻-Cu²⁺(Phen)₂, R₂ is a carboxylate, and R₃, R₄, R₅ and R₆ are H;
- R₁ is -Z-COO⁻-Cu²⁺(Phen)₂, R₃ is a carboxylate, and R₂, R₄, R₅ and R₆ are H;
- R₁ is -Z-COO⁻-Cu²⁺(Phen)₂, R₄ is a carboxylate, and R₂, R₃, R₅ and R₆ are H;
- R₁ is -Z-COO⁻-Cu²⁺(Phen)₂, R₅ is a carboxylate, and R₂, R₃, R₄ and R₆ are H;
- R₁ is -Z-COO⁻-Cu²⁺(Phen)₂, R₆ is a carboxylate, and R₂, R₃, R₄ and R₅ are H;
- R₁ and R₂ are -Z-COO⁻-Cu²⁺(Phen)₂, and R₃, R₄, R₅ and R₆ are ideally H;
- R₁ and R₃ are -Z-COO⁻-Cu²⁺(Phen)₂, and R₂, R₄, R₅ and R₆ are ideally H;
- R₁ and R₄ are -Z-COO⁻-Cu²⁺(Phen)₂, and R₂, R₃, R₅ and R₆ are ideally H;
- R₁ and R₅ are -Z-COO⁻-Cu²⁺(Phen)₂, and R₂, R₃, R₄ and R₆ are ideally H; and
- R₁ and R₆ are -Z-COO⁻-Cu²⁺(Phen)₂, and R₂, R₃, R₄ and R₅ are ideally H;
